# EUROPEAN PATENT SPECIFICATION

(11) **EP 1 407 661 B1**
(45) Date of publication and mention of the grant of the patent: **08.10.2008**
(21) Application number: 02738713.3
(22) Date of filing: 14.06.2002
(51) Int. Cl.: C12N 15/85, C12N 9/88, A01K 67/027, C12N 5/10, G01N 33/50

(54) **TRANSGENIC ANIMAL TRANSFORMED USING GREEN FLUORESCENT PROTEIN GENE**
MIT DEM GREEN FLUORESCENT PROTEIN -GEN TRANSFORMIERTES TRANSGENES TIER
ANIMAL TRANSGENIQUE TRANSFORME AU MOYEN D'UN GENE DE PROTEINE VERT FLUORESCENT

(30) Priority: 15.06.2001 JP 2001182612; 04.06.2002 JP 2002162552
(43) Date of publication of application: 14.04.2004
(73) Proprietor: Japan Science and Technology Agency, Kawaguchi-shi, Saitama 332-0012 (JP)
(72) Inventor: YANAGAWA, Yuchio, Okazaki-shi, Aichi 444-0878 (JP)
(74) Representative: Cresswell, Thomas Anthony
(86) International application number: PCT/JP2002/005949
(87) International publication number: WO 2002/102143

(56) References cited:
- ASADA HIDEO ET AL: "Mice lacking the 65 kDa isoform of glutamic acid decarboxylase (GAD65) maintain normal levels of GAD67 and GABA in their brains but are susceptible to seizures" BIOCHEMICAL AND BIOPHYSICAL RESEARCH COMMUNICATIONS, vol. 229, no. 3, 1996, pages 891-895, XP002341569 ISSN: 0006-291X
- KASH SHERA F ET AL: "Epilepsy in mice deficient in the 65-kda isoform of glutamic acid decarboxylase" PROCEEDINGS OF THE NATIONAL ACADEMY OF SCIENCES OF THE UNITED STATES OF AMERICA, vol. 94, no. 25, 9 December 1997 (1997-12-09), pages 14060-14065, XP002341339 ISSN: 0027-8424
- OLIVA ANTHONY A JR ET AL: "Novel hippocampal interneuronal subtypes identified using transgenic mice that express green fluorescent protein in GABAergic interneurons" JOURNAL OF NEUROSCIENCE, vol. 20, no. 9, 1 May 2000 (2000-05-01), pages 3354-3368, XP002341570 ISSN: 0270-6474
- FUCHS ELKE C ET AL: "Genetically altered AMPA-type glutamate receptor kinetics in interneurons disrupt long-range synchrony of gamma oscillation" PROCEEDINGS OF THE NATIONAL ACADEMY OF SCIENCES OF USA, NATIONAL ACADEMY OF SCIENCE. WASHINGTON, US, vol. 98, no. 6, 13 March 2001 (2001-03-13), pages 3571-3576, XP002193100 ISSN: 0027-8424
- GODWIN A R ET AL: "Detection of targeted GFP-Hox gene fusions during mouse embryogenesis" PROCEEDINGS OF THE NATIONAL ACADEMY OF SCIENCES OF USA, NATIONAL ACADEMY OF SCIENCE. WASHINGTON, US, vol. 95, October 1998 (1998-10), pages 13042-13047, XP002960122 ISSN: 0027-8424
- HADJANTONAKIS ANNA-KATERINA ET AL: "The color of mice: In the light of GFP-variant reporters" HISTOCHEMISTRY AND CELL BIOLOGY, vol. 115, no. 1, January 2001 (2001-01), pages 49-58, XP002341571 ISSN: 0948-6143
- TUCKER KERRY LEE: "In vivo imaging of the mammalian nervous system using fluorescent proteins" HISTOCHEMISTRY AND CELL BIOLOGY, vol. 115, no. 1, January 2001 (2001-01), pages 31-39, XP002341572 ISSN: 0948-6143
- TAMAMAKI NOBUAKI ET AL: "Green fluorescent protein expression and colocalization with calretinin, parvalbumin, and somatostatin in the GAD67-GFP knock-in mouse." JOURNAL OF COMPARATIVE NEUROLOGY, vol. 467, no. 1, 1 December 2003 (2003-12-01), pages 60-79, XP008051325 ISSN: 0021-9967
- JAPANESE JOURNAL OF NEUROPSYCHOPHARMACOLOGY vol. 7, no. 4, 1985, pages 255 - 264, XP002960271
- MASAHITO IKAWA: 'Green fluorescent protein as a marker in transgenic mice' DEVELOP. GROWTH DIFFER. vol. 37, no. 4, 1995, OSAKA, pages 455 - 459, XP002086829
- MASARU OKABE ET AL: 'Green Fluorrescent Protein as a Novel Vital Marker in Transgenic Mouse' J. REPROD. DEV. vol. 43, no. 6, 1997, pages J19 - J25, XP002960273
- HIDEO ASADA ET AL: 'Cleft palate and decreased brain GAMMA-aminobutyric acid in mice lacking the 67-kDa isoform of glutamic acid decarboxylase' PROC. NATL. ACAD. SCI. USA (NEUROBIOLOGY). vol. 94, no. 12, June 1997, pages 6496 - 6499, XP002960274
- BRIAN G. CONDIE ET AL: 'Cleft palate in mice with a targeted mutation in the GAMMA-aminobutyric acid-producing enzyme glutamic acid decarboxylase 67.' PROC. NATL. ACAD. SCI. USA (GENETICS). vol. 94, no. 21, October 1997, pages 11451 - 11455, XP002960275
- EMA M. ET AL.: 'Deletion of selection cassette but not cis-acting elements in targeted Flk1-LacZ allele reveals Flk1 expression in multipotent mesodermal progenitors' BLOOD 15 September 2005, pages 1 - 28
- TANAKA M. ET AL.: 'Parental origin-specific expression of Mash2 is established at the time of implantation with its imprinting mechanism highly resistant to genome-wide methylation' MECHANISMS OF DEVELOPMENT vol. 87, 1999, pages 129 - 142
- KAKIZAKI T. ET AL.:'The role of GABAergic system in ventral body wall formation', Program number PS3A-C034, 21 July
- TSUNEKAWA N. ET AL.: 'Development of spontaneous mouth/tongue movement and related neural activity, and their repression in fetal mice lacking glutamate decarboxylase 67' NEUROSCIENCE vol. 21, 2005, pages 173 - 178
- KUWANA S. ET AL.: 'Disturbance of neural respiratory control in neonatal mice lacking GABA synthesizing enzyme 67-kDa isoform of glutamic acid decarboxylase' NEUROSCIENCE vol. 120, 2003, pages 861 - 870
- MOIKE T. ET AL.: 'Universal GFP reporter for the study of vascular development' GENESIS vol. 28, 2000, pages 75 - 81
- KATAROVA Z. ET AL.: 'Regulation of cell-type specific expression of Lacz by 5'-flanking region of mouse GAD67 gene in the central nervous system of transgenic mice' EUROPEAN JOURNAL OF NEUROSCIENCE vol. 10, 1998, pages 989 - 999
- YANAGAWA Y. ET AL.: 'Cleft-palate and omphalocele in vesicular GABA transporter knockout mice and glutamate decarboxylase 67 knockout mice' 7TH IBRO WORLD CONGRESS OF NEUROSCIENCE 12 July 2007, page 280
- KUWANA S-I. ET AL.: 'Electrophysiological and morphological characteristics of GABAergic respiratory neurons in the mouse pre-Bötzinger complex' EUROPEAN JOURNAL OF NEUROSCIENCE vol. 23, 2006, pages 667 - 674

## Description

### Technical Field

The present invention relates to a transgenic (knock-in) animal transformed using a green fluorescent protein gene, a method for preparing the transgenic animal, and a model animal with cleft palate.

### Background of the Invention

Brain is constituted of assemblies of neuronal networks composed of excitatory and inhibitory neurons. It has been known that as a main inhibitory transmitter in the central nervous system, γ-aminobutyric acid (GABA) plays a major role in brain functions such as wakefulness, sleep, circadian rhythm, learning, motion and sensory information. Furthermore, it has been reported that GABA is associated with psychoneurotic diseases such as epilepsy and alcoholic psychosis and is also associated with psychotic symptoms such as anxiety and depression. Thus, the elucidation of (1) the action mechanism of GABA and (2) the function of the GABAergic neuron with GABA as a transmitter can make a contribution to the understanding of brain functions as well as the elucidation of the pathogenesis of the diseases and the establishment of their therapeutic methods.

GABA is synthesized from glutamic acid by glutamate decarboxylase (GAD). It has been found that the enzyme includes two types of isozymes (GAD65 and GAD67). It has been known that GAD is the most specific marker of the GABAergic neurons and that these neurons acquire their GABAergic phenotype progressively as GAD gene expression levels increase during development. (1. Erlander, M.G., Tillakaratne, N.J.K, Feldblum, S., Patel, N. & Tobin, A.J. (1991) Two genes encode distinct glutamate decarboxylases. Neuron 7, 91-100; 2. Esclapez, M., Tillakaratne, N. J., Kaufman, D.L., Tobin, A.J., & Houser, C.R. (1994). Comparative localization of two forms of glutamic acid decarboxylase and their mutant mRNAs in rat brain supports the concept of functional differences between the forms. J. Neurosci. 14, 1834-1855).

Cleft plate is one of the most common congenital defects, which involves a fissure at lip and upper jaw (plate). In man palate is formed in gestational age of 6 to 12 weeks by fusion between median palatal protrusion derived from medial nasal fold and lateral palatal protrusion derived from upper jaw protrusion, as if they were closed with a zipper. It is believed that a disorder happening during the period causes cleft palate. It is said that cleft palate occurs at a ratio of one out of 1800 to 1000 individuals and is one of the most congenital abnormality in Japan. It is also said that both environmental factors and genetic factors are responsible for the cause of the occurrence. However, the details are not yet elucidated.

As the therapeutic method of cleft palate, only surgery is now applicable. Therefore, investigations over the cause of cleft palate are needed.

Asada et al. (1997) PNAS USA 94, 6496-99 discloses the observation of cleft palate and decreased brain γ-aminobutyric acid in mice lacking the 67-kDa isoform of glutamic acid decarboxylase.

Condie et al. (1997) PNAS USA 94, 11451-5 discloses the observation of cleft palate in mice with a targeted mutation in the γ-aminobutyric acid-producing enzyme glutamic acid decarboxylase 67.

Asada et al. (1996) Biochemical and Biophysical Research Communications 229, 891-5 discloses that mice lacking the 65kDa isoform of glutamic acid decarboxylase maintain normal levels of GAD67 and GABA in their brains but are susceptible to seizures.

Kash et al. (1997) PNAS USA 94, 14060-5 discloses the observation of epilepsy in mice deficient in the 65-kDa isoform of glutamic acid decarboxylase.

Oliva Jr et al (2000) Journal of Neuroscience 20, 3354-68 discloses novel hippocampal interneuronal subtypes identified using transgenic mice that express green flourescent protein in GABAergic interneurons.

Fuchs et al (2001) PNAS USA, 98, 3571-6 discloses that genetically altered AMPA-type glutamate receptor genetics in interneurons disrupt long-range synchrony of gamma oscillation.

Godwin et al. (1998) PNAS USA 95, 13042-7 discloses detection of targeted GFP-Hox gene fusions during mouse embryo genesis.

### Disclosure of the Invention

It was very difficult to exactly identify the GABAergic neurons in vivo and analyze their functions such as electrophysiological characteristics. Because the GABAergic neurons are generally scattered in the central nervous system and the number of them is relatively small. Based on the background, for the purpose of visualizing the GABAergic neurons in vivo, the present inventor developed a transgenic mouse (gene-modified mouse), obtained by knocking-in the gene encoding the fluorescent molecule (green fluorescent protein (GFP)) derived from luminous Aequoria victoria in the GAD76 locus, and named the transgenic animal GAD67-GFP knock-in mouse. Consequently, the invention has been achieved.

The inventor successfully achieved the preparation of a transgenic mouse where the GFP gene is inserted into the same reading frame as that of exon 1 of the glutamate decarboxylase 67 gene in order to obtain a mouse where the GABAergic neurons can be visualized in vivo. Additionally, the inventor found the resulting mouse had congenital cleft palate.

Specifically, the invention provides a transgenic mouse comprising a nucleic acid sequence encoding a green fluorescent protein, wherein said nucleic acid sequence is inserted in frame with exon 1 of an endogenous glutamate decarboxylase 67 gene. The transgenic mouse of the invention is very useful as a model animal of cleft palate.

Additionally, the invention relates to a method for preparing a transgenic mouse as a heterozygote; including a step of integrating a target vector carrying the green fluorescent protein gene into an embryo stem cell to induce homologous recombination, bringing the resulting homologous recombinant back in the embryo to prepare a chimera mouse and allowing the chimera mouse to mate with the wild type mouse; and a method for preparing a transgenic mouse as a homozygote including a step of allowing the transgenic mouse thus obtained as the heterozygote to mate with one another.

Still additionally, the invention relates to a mouse embryo stem cell obtained by homologous recombination with a targeting vector where the green flourescent protein gene is inserted in the same reading frame as that of an exon of the glutamate decarboxylase gene.

### Brief Description of the Drawings

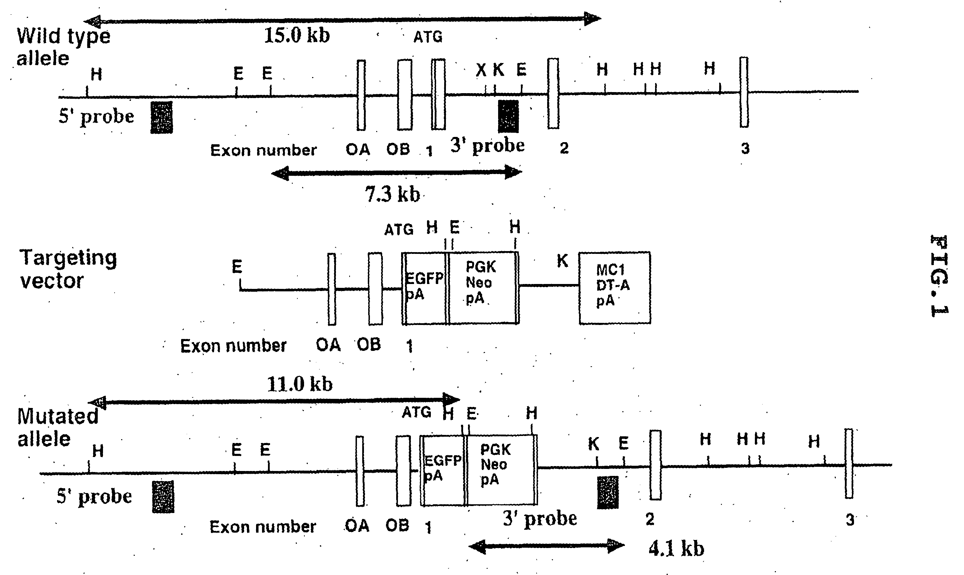

Fig. 1 shows the organization of the mouse GAD67 gene (wild-type allele), the targeting vector, and the GAD67 gene (mutant allele) after homologous recombination.
   The length of DNA as indicated by an arrow with points at both ends in the figure shows the size of a band, and the filled box represents the region of a probe by Southern blot analysis.

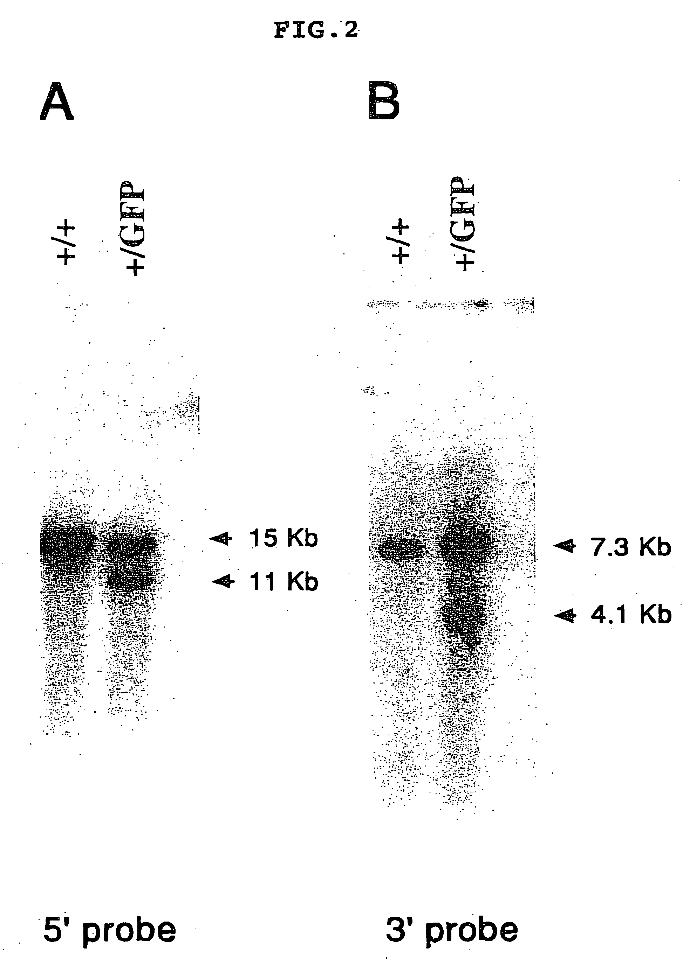

Fig. 2 is a photograph substituted for a drawing showing the results of Southern blot analysis of ES cell genome DNA. Fig. 2 (A) shows the results of hybridization with ES cell genome DNAs derived from the wild type (+/+) and the heterozygote (+/GFP), enzymatically treated with HindIII, using 5' probe. In the wild type, only one band of 15-kb is observed. In the heterozygote, a band of 11-kb from homologous recombination is observed, in addition to the 15-kb band. Fig. 2 (B) shows the results of hybridization with ES cell genome DNAs derived from the wild type (+/+) and the heterozygote (+/GFP), enzymatically treated with EcoRI, using 3' probe. In the wild type, only one band of 7.3-kb is observed. In the heterozygote, a band of 4.1-kb from homologous recombination is observed, in addition to the 7.3-kb band.

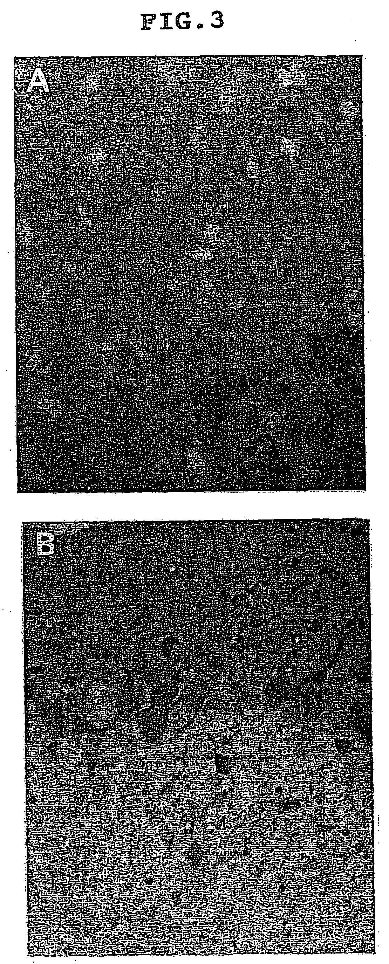

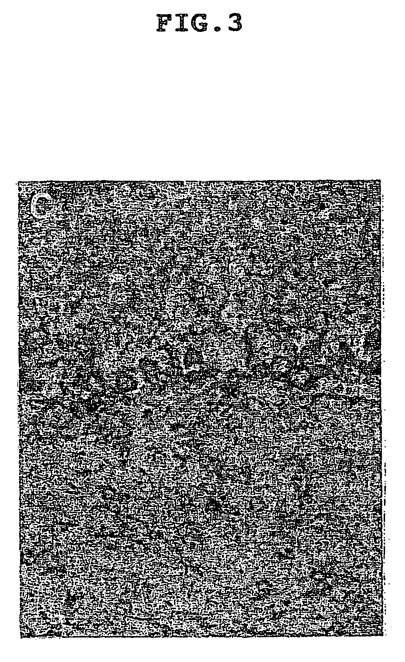

Fig. 3 is a photograph substituted for a drawing showing histological sections of the GAD67-GFP knock-in mouse cerebellar cortex. Fig. 3 (A) is obtained on the basis of self-fluorescence; (B) is obtained by immunohistological staining using anti-GFP antibody by the conventional method; and (C) is obtained by immunohistological staining using anti-GAD67 antibody. In any of the photographs, the upper part shows the molecular layer; the middle part shows Purkinje cell layer; and the lower part shows the granular layer.

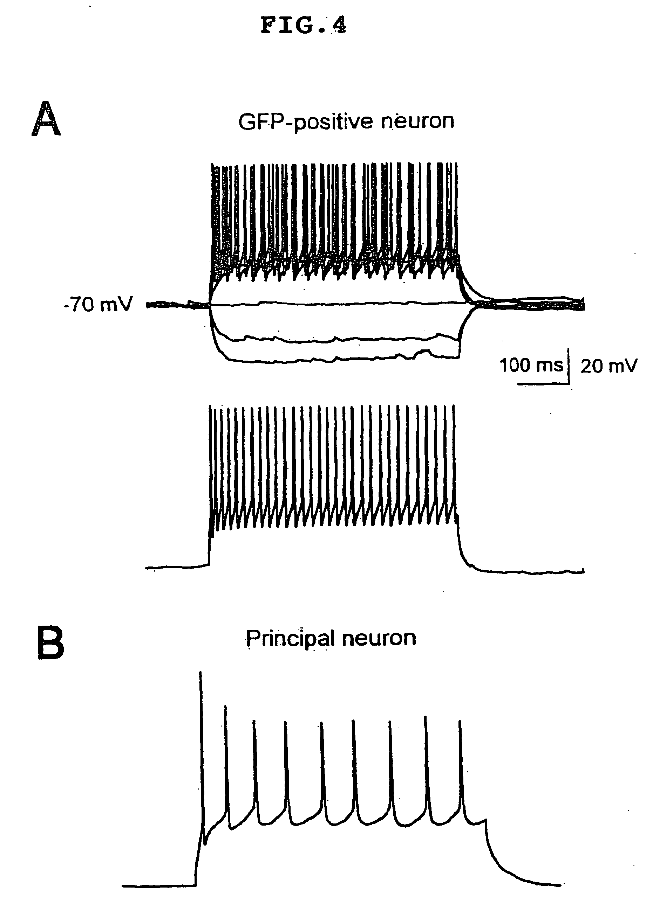

Fig. 4 is a chart showing the results of the electrophysiological analysis of the cell in the amygdala with the whole-cell patch clamp method. Fig. 4 (A) is a record from the GFP-positive cell (GABAergic neuron) of the GAD67-GFP knock-in mouse. Fg.4 (B) is a record from the principal cell (non-GABAergic neuron) of the wild type mouse. The firing rate of the GFP-positive cell is much higher than that of the principal cell.

Glutamate decarboxylase (GAD) includes isozymes such as GAD67 and GAD65.

The green fluorescent protein is a protein isolated from luminous Aequoria victoria, which has a property to be luminous in green color under irradiation of ultraviolet ray or blue light. As the green fluorescent protein of the invention, any protein from any origin can be used. A luminous gene having fluorescence in the visible region is applicable to the method of the invention.

The transgenic mouse of the invention can be prepared by various methods in the art.

Typical examples thereof include a method of microinjecting an appropriate vehicle carrying the green fluorescent protein gene in the pronucleus of a fertilized mouse egg and bringing the survived cell back into the uterine tube of a psudopregnant parent; a method of integrating a targeting vector carrying the green fluorescence protein gene in a mouse embryo stem cell (targeting construct) for inducing homologous recombination, screening the resulting homologous recombinant, bringing the homologous recombinant back in the blastcyst to form a chimera embryo, transferring the chimera embryo in the uterine of a psudopregnant parent to prepare a chimera mouse, and allowing the chimera mouse to mate with the wild type mouse to prepare a transgenic mouse as a heterozygote; and a method for preparing a transgenic including a step of allowing the thus obtained transgenic mouse as a heterozygote to mate with one another.

In order to screen and concentrate the resulting homologous recombinant, the targeting vector preferably contains positive selection markers such as the neomycin-resistant gene, the hygromycin-resistant gene, and the puromycin-resistant gene, and negative selection markers such as the diphtheria toxin A gene and the herpes simplex virus thymidine kinase gene, in addition to the green fluorescent protein gene.

The green fluorescent protein gene is inserted in the same reading frame as that of exon 1 of the glutamate decarboxylase 67 gene. Therefore, the green fluorescent protein gene is expressed simultaneously with the expression of the glutamate decarboxylase 67 gene.

The transgenic mouse thus obtained, where the foreign gene is inserted in the same reading frame as that of an exon 1 of the glutamate decarboxylase 67 gene, shows cleft palate and is found to be a useful model animal of the diseased condition. Cleft palate is a congenital abnormality at a relatively high incidence in Japan. Investigations about the cause of the disease are now needed and the development of a therapeutic method thereof is expected.

Thus, the invention provides a novel transgenic mouse useful as a model animal with cleft palate.

By the use of the model mouse of the invention, not only a screening (experiment) can be done for investigating the cause of cleft palate, but a screening of a method of drugs or gene therapy for therapeutic methods of cleft palate, or a screening for the determination of the time to treat the disease can be done using the model mouse of the invention. For such screening methods of the invention using the model mouse of the invention, various known screening methods are applicable.

### Examples

The invention is now described in detail with reference to the following Examples. However, these Examples never limit the scope of the invention in any sense.

### Example 1 Preparation of targeting construct

A targeting construct was prepared, using the structure of the mouse GAD67 gene and the gene clone thereof as isolated and identified by the inventor (Yanagawa Y., et al., Biochem. J., 326, 573-578 (1997); Asada, H., et al., Proc. Natl. Acad. Sci. USA, 94, 6496-6499 (1997)). This is shown in Fig. 1. Specifically, a targeting construct was placed the enhanced GFP (EGFP) gene (purchased from Clontech) into exon 1 of the mouse GAD67 gene, in-frame with the initiation codon (ATG). As the positive selection marker, the neomycin-resistant gene (PGK-Neo-pA) was inserted. As the negative selection marker, the diphtheria toxin A gene (MCl-DT-A-pA) was inserted (Yanagawa, Y., et al., Transgenic Res., 8, 215-221 (1999)). The length of the homologous region of the GAD67 gene used as the targeting vector was 5.0 Kb at the 5' side of the EGFP gene and 1.6 Kb at the 3' side of the neomycin-resistant gene.

### Example 2 Preparation of transgenic mouse (GAD67-GFP knock-in mouse)

After the above targeting construct was introduced in mouse ES cells, 251 G418-resistant ES clones were analyzed. As a result of screening by PCR and Southern blot analysis, 13 ES clones were identified where GAD67-GFP was knocked-in by homologous recombination. The efficiency of the homologous recombination was 5.2 %. The result of Southern blot analysis is shown in Fig. 2. These clones were injected in mouse embryos at the 8-cell stage, to obtain a chimera mouse. As the results of the mating of the chimera mouse with the wild type mouse, it was identified under the observation of their hair color that all the 13 newborn mice were the mice derived from the ES cells. Further, 7 out of the 13 newborn mice were the GAD67-GFP knock-in mouse, as identified by genotyping by PCR.

### Example 3 Analysis of GAD67-GFP knock-in mouse

From the brain of the GAD67-GFP knock-in mouse, a specimen of the cerebellum was prepared for observation of the self-fluorescence of GFP and for examination of the immunohistochemistry using an anti-GFP antibody and an anti-GAD67 antibody. Fig. 3 shows the results of these observations and examinations using a cerebellar cortex specimen. As the cell where GFP fluorescence is observed, numerous small cells in the molecular layer, large cells in the Purkinje cell layer and a small number of cells scattered in the granular layer were identified. The cell where GFP fluorescence was observed had the same distribution of the GABAergic neuron as shown in a great number of reports so far. Additionally, even the findings based on the immunohistochemistry using an anti-GFP antibody and an anti-GAD67 antibody suggest that the immunopositive cells have the same distribution as that of the GABAergic neuron. The results described above indicate that the cell with the self-fluorescence of GFP is the GABAergic neuron.

The cell in the amygdala was electrophysiologically analyzed using the whole cell patch clamp method (Edwards FA., et al., Pflugers Arch 414, 600-612 (1989); Kawaguchi Y., et al., J. Neurophysiol., 70, 387-396 (1993)) (Fig. 4). As a result of recording from the GFP-positive cell in the amygdala of the GAD67-GFP knock-in mouse, the firing rate was observed high. On the other hand, as a result of recording from the principal cell of the wild type mouse, it was observed that the frequency of the action potential was not so high compared with that of record from the GFP-positive neurons. The results described above show the firing patterns characteristic to those observed in the GABAergic neuron and the principal cell and support that in view of the function, the GFP positive cell is the GABAergic neuron. Thus, it was concluded that the gene-modified mouse was useful as the analysis of the function of the GABAergic neuron.

The GAD67-GFP knock-in mouse prepared in accordance with the invention can be used for (1) the electrophysiological analysis of the GABAergic neuron, (2) the morphological analysis of the GABAergic neuron, (3) the analysis of the molecular biology intrinsic to the GABAergic neuron and (4) the analysis of the in vivo expression dynamics of the GAD67 gene, and is expected to be used as a resource required for brain research.

As a result of mating the heterozygous GAD67 GFP knockin mice (+/GFP) with one another, a homozygote (GFP/GFP) was obtained. Cleft palate was found in the homozygote. The results correspond to the results from the GAD67 knockout mouse as reported so far. As the cause of cleft palate, a number of causes have been suggested, however, it is known that GABA is involved in the occurrence of a certain type of cleft palate. Thus, the GAD67-GFP knock-in mouse will be a model animal for elucidating diseases in which GABA is involved, such as cleft palate. Then, the GAD67-GFP knock-in mouse may be useful for elucidating the action mechanism of GABA. Further, the β-cell of the pancreas Langerhans island secretes insulin and is known to be GAD67-positive. Therefore, it is expected the β-cell of the Langerhans island will possibly be visualized in vivo in the GAD67-GFP knock-in mouse, which may be useful for elucidating the mechanism of the onset of diabetes mellitus.

### Example 4 Cleft palate of the model animal of the invention

The birth rate in the model animal of palatoschisis of the invention was examined. The homozygote of the GAD67-GFP knock-in mouse developed cleft palate, and died on the date of birth. Mating the heterozygous GAD67-GFP knock-in mice with one another produced 33 newborns, but 9 out of the newborns died on their dates of birth. (Death rate: 27%).

### Industrial Applicability

Cells where the glutamate decarboxylase is expressed in the GAD67-GFP knock-in mouse of the invention can be visualized on the basis of the fluorescence, and the expression of the glutamate decarboxylase can be observed in more details, compared with the conventional methods. Thus, the GAD67-GFP knock-in mouse of the invention can be used for (1) the elucidation of the action mechanism of GABA and (2) for the elucidation of the function and morphology of the GABAergic neuron.

Further, the transgenic animal of the invention shows cleft palate recognized as a congenital abnormality and is therefore useful as a model animal of cleft palate.

## Claims

1. A transgenic mouse comprising a nucleic acid sequence encoding a green fluorescent protein, wherein said nucleic acid sequence is inserted in frame with exon I of an endogenous glutamate decarboxylase 67 gene.

## Patentansprüche

1. Transgene Maus, umfassend eine Nukleinsäuresequenz, die für ein grün fluoreszierendes Protein codiert, wobei die Nukleinsäuresequenz in-frame mit Exon 1 eines endogenen Glutamatdecarboxylase 67-Gens inseriert ist.

## Revendications

1. Souris transgénique comprenant une séquence d'acides nucléiques codant pour une protéine fluorescente verte, dans laquelle ladite séquence d'acides nucléiques est insérée dans le cadre de lecture de l'exon 1 d'un gène d'une glutamate decarboxylase 67 endogène.
